# EUROPEAN PATENT SPECIFICATION

(11) **EP 1 856 243 B1**
(45) Date of publication and mention of the grant of the patent: **21.04.2010**
(21) Application number: 06715685.1
(22) Date of filing: 09.03.2006
(51) Int. Cl.: C12P 13/08, C12P 13/10, C12P 13/12, C12P 13/20

(54) **PROCESS FOR PRODUCING AN L-AMINO ACID EMPLOYING A BACTERIUM OF THE ENTEROBACTERIACEAE FAMILY WITH ATTENUATED LEUO EXPRESSION**
VERFAHREN ZUR HERSTELLUNG EINER L-AMINOSÄURE UNTER VERWENDUNG EINES BAKTERIUMS DER FAMILIE ENTEROBACTERIACEAE MIT ABGESCHWÄCHTER LEUO-EXPRESSION
PROCÉDÉ DE PRODUCTION D'UN L-ACIDE AMINÉ EN UTILISANT D'UNE BACTERIE ISSUE DE LA FAMILLE DES ENTEROBACTERIACEAE PRÉSENTANT UNE EXPRESSION ATTENUÉE DU GÈNE LEUO

(30) Priority: 10.03.2005 RU 2005106346; 06.10.2005 US 723925 P
(43) Date of publication of application: 21.11.2007
(73) Proprietor: Ajinomoto Co., Inc., Tokyo 104-8315 (JP)
(72) Inventor: RYBAK, Konstantin, Vyacheslavovich, Moscow 117149 (RU); VOROSHILOVA, Elvira, Borisovna, Moscow 117646 (RU); LEONOVA, Tatyana, Viktorovna, Moscow 123481 (RU); KOZLOV, Yury, Ivanovich, Moscow 117574 (RU)
(74) Representative: HOFFMANN EITLE
(86) International application number: PCT/JP2006/305194
(87) International publication number: WO 2006/103936

(56) References cited:
- KLAUCK, E. ET AL.: "The LysR-like regulator LeuO in Escherichia coli is involved in the translational regulation of rpoS by affecting the expression of the small regulatory DsrA-RNA" MOLECULAR MICROBIOLOGY, vol. 25, no. 3, August 1997 (1997-08), pages 559-569, XP002405299
- MAJUMDER, A. ET AL.: "LeuO Expression in Response to Starvation for Branched-chain Amino Acids" JOURNAL OF BIOLOGICAL CHEMISTRY, vol. 276, no. 22, 1 June 2001 (2001-06-01), pages 19046-19051, XP002405300 cited in the application

## Description

### Technical Field

The present invention relates to the microbiological industry, and specifically to a method for producing an L-amino acid using a bacterium of the *Enterobacteriaceae* family, which has been modified to attenuate expression of the *leuO* gene.

### Background Art

The LeuO protein, which is a transcriptional activator of the *leuABCD* operon, belongs to the LysR family (Henikoff, S. et al, Proc. Natl. Acad. Sci. USA, 85, 6602-6606 (1988)).

It has been shown that the *leuO* gene causes the *hns*⁻complementing phenotype, which is a mutation in the histone-like protein H-NS. Namely, overexpression of the *leuO* gene from a multicopy plasmid drastically reduces production of CadC, the essential activator for inducing the *cadA* gene, which encodes biodegradative lysine decarboxylase (Shi, X. and Bennett, G.N., J. Bacteriol., 177, 3, 810-814 (1995)).

It has also been shown that the LeuO regulatory protein is involved in the transcriptional regulation of the *rpoS* gene by affecting the expression of the small regulatory DsrA-RNA (Klauck, E. et al, Mol. Microbiol., 25, 3, 559-569 (1997)).

The *leuO* gene product has a latent ability to relieve *bgl* silencing in *Escherichia coli,* where the *bgl* operon is involved in the utilization of certain β-glucosides, such as salicin and arbutin (Ueguchi, C. et al, J. Bacteriol., 180, 1, 190-193 (1998)).

A conditional *leuO* expression was found when bacteria enter the stationary phrase and was shown to be guanosine 3', 5'-bispyrophosphate-dependent. Multiple physiological events, including the stringent response, are induced upon the increase of the bacterial stress signal, guanosine 3', 5'-bispyrophosphate. It was shown that LeuO is indispensable for growth resumption following a 2-h growth arrest caused by starvation for branched-chain amino acids in an *E. coli* K-12 *relA1* strain, which supports the idea that LeuO has a functional role in the bacterial stringent response (Majumder, A et al, J. Biol. Chem., 276, 22, 19046-19051 (2001)).

But currently, there have been no reports of inactivating the *leuO* gene for the purpose of producing L-amino acids.

### Disclosure of the Invention

Objects of the present invention include enhancing the productivity of L-amino acid producing strains, and providing a method for producing an L-amino acid using these strains.

The above objects were achieved by finding that attenuating expression of the *leuO* gene can enhance production of L-amino acids, such as L-threonine, L-lysine, L-methionine, L-asparagine, L-aspartic acid, and L-arginine.

The present invention provides the use of a bacterium of the *Enterobacteriaceae* family having an increased ability to produce amino acids, such as L-threonine, L-lysine, L-methionine, L-asparagine, L-aspartic acid, and L-arginine.

It is an object of the present invention to provide the use of an L-amino acid producing bacterium of the *Enterobacteriaceae* family, wherein the bacterium has been modified to attenuate expression of the *leuO* gene.

It is a further object of the present invention to provide the use of the bacterium as described above, wherein the expression of the *leuO* gene is attenuated by inactivation of the *leuO* gene.

It is a further object of the present invention to provide the use of the bacterium as described above, wherein the bacterium belongs to the genus *Escherichia.*

It is a further object of the present invention to provide the use of the bacterium as described above, wherein the bacterium belongs to the genus *Pantoea.*

It is a further object of the present invention to provide a method for producing an L-amino acid comprising:
- cultivating the bacterium as described above in a medium so to produce and excrete said L-amino acid into the medium, and
- collecting said L-amino acid from the medium, wherein said L-amino acid is selected from the group consisting of L-threonine, L-lysine, L-methionine, L-asparagine, L-aspartic acid, and L-arginine.

The present invention is described in detail below.

### Detailed Description of the preferred Embodiments

### 1. Bacterium for use in the present invention

The bacterium for use in the present invention is an L-amino acid producing bacterium of the *Enterobacteriaceae* family, wherein the bacterium has been modified to attenuate expression of the *leuO* gene.

In the present disclosure, "L-amino acid producing bacterium" means a bacterium, which has an ability to produce and excrete an L-amino acid into a medium, when the bacterium is cultured in the medium.

The phrase "L-amino acid-producing bacterium" as used herein also means a bacterium which is able to produce and cause accumulation of an L-amino acid in a culture medium in an amount larger than a wild-type or parental strain of *E*. *coli,* such as *E. coli* K-12, and preferably means that the microorganism is able to cause accumulation in a medium of an amount not less than 0.5 g/L, more preferably not less than 1.0 g/L of the target L-amino acid. The term "L-amino acids" includes L-alanine, L-arginine, L-asparagine, L-aspartic acid, L-cysteine, L-glutamic acid, L-glutamine, glycine, L-histidine, L-isoleucine, L-leucine, L-lysine, L-methionine, L-phenylalanine, L-proline, L-serine, L-threonine, L-tryptophan, L-tyrosine, and L-valine.

The term "aromatic L-amino acid" includes L-phenylalanine, L-tyrosine, and L-tryptophan. The term "non-aromatic L-amino acid" includes L-threonine, L-lysine, L-cysteine, L-methionine, L-leucine, L-isoleucine, L-valine, L-histidine, glycine, L-serine, L-alanine, L-asparagine, L-aspartic acid, L-glutamine, L-glutamic acid, L-proline, and L-arginine.

The *Enterobacteriaceae* family includes bacteria belonging to the genera *Escherichia, Enterobacter, Erwinia, Klebsiella, Pantoea, Photorhabdus, Providencia, Salmonella, Serratia, Shigella, Morganella Yersinia,* etc.. Specifically, those classified into the *Enterobacteriaceae* according to the taxonomy used in the NCBI (National Center for Biotechnology Information) database (http://www.ncbi.nlm.nih.gov/Taxonomy/Browser/wwwtax.cgi?id=91347) can be used. A bacterium belonging to the genus *Escherichia* or *Pantoea* is preferred.

The phrase "a bacterium belonging to the genus *Escherichia"* means that the bacterium is classified into the genus *Escherichia* according to the classification known to a person skilled in the art of microbiology. Examples of a bacterium belonging to the genus *Escherichia* as used in the present invention include, but are not limited to, *Escherichia coli* (*E. coli*).

The bacterium belonging to the genus *Escherichia* that can be used in the present invention is not particularly limited, however for example, bacteria described by Neidhardt, F.C. et al. (Escherichia coli and Salmonella typhimurium, American Society for Microbiology, Washington D.C., 1208, Table 1) are encompassed by the present disclosure.

The phrase "a bacterium belonging to the genus *Pantoea"* means that the bacterium is classified as the genus *Pantoea* according to the classification known to a person skilled in the art of microbiology. Some species of *Enterobacter agglomerans* have been recently re-classified into *Pantoea agglomerans, Pantoea ananatis, Pantoea stewartii* or the like, based on nucleotide sequence analysis of 16S rRNA, etc. (Int. J. Syst. Bacteriol., 43,162-173 (1993))

The phrase "bacterium has been modified to attenuate expression of the *leuO* gene" means that the bacterium has been modified in such a way that a modified bacterium contains a reduced amount of the LeuO protein as compared with an unmodified bacterium, or the modified bacterium is unable to synthesize the LeuO protein. The phrase "bacterium has been modified to attenuate expression of the *leuO* gene" also means that the target gene is modified in such a way that the modified gene encodes a mutant LeuO protein which has a decreased activity.

The phrase "inactivation of the *leuO* gene" means that the modified gene encodes a completely non-functional protein. It is also possible that the modified DNA region is unable to naturally express the gene due to the deletion of a part of the gene, the shifting of the reading frame of the gene, the introduction of missense/nonsense mutation(s), or the modification of an adjacent region of the gene, including sequences controlling gene expression, such as a promoter, enhancer, attenuator, ribosome-binding site, etc.

The *leuO* gene encodes the LeuO protein (synonym - B0076), which is a transcriptional activator of the *leuABCD* operon. The *leuO* gene of *E*. *coli* (nucleotides 84191 to 85312 in the GenBank accession number NC_000913.2; gi:49175990; SEQ ID NO: 1) is located between the *leuL* gene coding for the *leuABCD* operon leader peptide and the *ilvI* gene on the chromosome *of E. coli* K-12. The nucleotide sequence of the *leuO* gene and the amino acid sequence of LeuO encoded by the *leuO* gene are shown in SEQ ID NO: 1 and SEQ ID NO: 2, respectively.

Since there may be some differences in DNA sequences between the genera or strains of the *Enterobacteriaceae* family, the *leuO* gene to be inactivated on the chromosome is not limited to the gene shown in SEQ ID NO:1, but may include genes homologous to SEQ ID NO:1 encoding a variant protein of the LeuO protein. The phrase "variant protein" as used in the present disclosure means a protein which has changes in the sequence, whether they are deletions, insertions, additions, or substitutions of amino acids, but still maintains the activity of the product as the LeuO protein which activates the *leuABCD* operon. The number of changes in the variant protein depends on the position or the type of amino acid residues in the three dimensional structure of the protein. It may be 1 to 30, preferably 1 to 15, and more preferably 1 to 5 in SEQ ID NO: 2. These changes in the variants can occur in regions of the protein which are not critical for the function of the protein. This is because some amino acids have high homology to one another so the three dimensional structure or activity is not affected by such a change. These changes in the variant protein can occur in regions of the protein which are not critical for the function of the protein. Therefore, the protein variant encoded by the *leuO* gene may have a homology of not less than 80 %, preferably not less than 90%, and most preferably not less than 95 %, with respect to the entire amino acid sequence shown in SEQ ID NO: 2, as long as the ability of the LeuO protein to activate the *leuABCD* operon or complement *hns* mutation prior to inactivation is maintained.

Homology between two amino acid sequences can be determined using the well-known methods, for example, the computer program BLAST 2.0, which calculates three parameters: score, identity and similarity.

Moreover, the *leuO* gene may be a variant which hybridizes under stringent conditions with the nucleotide sequence shown in SEQ ID NO: 1, or a probe which can be prepared from the nucleotide sequence, provided that it encodes a functional LeuO protein prior to inactivation. "Stringent conditions" include those under which a specific hybrid, for example, a hybrid having homology of not less than 60%, preferably not less than 70%, more preferably not less than 80%, still more preferably not less than 90%, and most preferably not less than 95%, is formed and a non-specific hybrid, for example, a hybrid having homology lower than the above, is not formed. For example, stringent conditions are exemplified by washing one time or more, preferably two or three times at a salt concentration of 1 × SSC, 0.1% SDS, preferably 0.1 × SSC, 0.1% SDS at 60ºC. Duration of washing depends on the type of membrane used for blotting and, as a rule, should be what is recommended by the manufacturer. For example, the recommended duration of washing for the Hybond^{™} N+ nylon membrane (Amersham) under stringent conditions is 15 minutes. Preferably, washing may be performed 2 to 3 times. The length of the probe may be suitably selected depending on the hybridization conditions, and is usually 100 bp to 1 kbp.

Expression of the *leuO* gene can be attenuated by introducing a mutation into the gene on the chromosome so that intracellular activity of the protein encoded by the gene is decreased as compared with an unmodified strain. Such a mutation on the gene can be replacement of one base or more to cause amino acid substitution in the protein encoded by the gene (missense mutation), introduction of a stop codon (nonsense mutation), deletion of one or two bases to cause a frame shift, insertion of a drug-resistance gene, or deletion of a part of the gene or the entire gene (J. Biol. Chem., 1997, 272 (13): 8611-8617, J. Antimicrobial Chemotherapy, 2000, 46: 793-79). Expression of the *leuO* gene can also be attenuated by modifying an expression regulating sequence such as the promoter, the Shine-Dalgamo (SD) sequence, etc. (WO95/34672, Biotechnol. Prog. 1999, 15, 58-64).

For example, the following methods may be employed to introduce a mutation by gene recombination. A mutant gene encoding a mutant protein having a decreased activity is prepared, and a bacterium to be modified is transformed with a DNA fragment containing the mutant gene. Then the native gene on the chromosome is replaced with the mutant gene by homologous recombination, and the resulting strain is selected. Such gene replacement using homologous recombination can be conducted by the method employing a linear DNA, which is known as "Red-driven integration" (Proc. Natl. Acad. Sci. USA, 2000, 97 (12): 6640-6645, WO2005/010175), or by the method employing a plasmid containing a temperature-sensitive replication control region (Proc. Natl. Acad. Sci. USA, 2000, 97 (12): 6640-6645, U.S. Patent Nos. 6,303,383 and 5,616,480). Furthermore, introduction of a site-specific mutation by gene replacement using homologous recombination as set forth above can also be performed by using a plasmid lacking the ability to replicate in the host.

Expression of the gene can also be attenuated by insertion of a transposon or an IS factor into the coding region of the gene (U.S. Patent No. 5,175,107), or by conventional methods, such as mutagenesis treatment using UV irradiation or nitrosoguanidine (N-methyl-N'-nitro-N-nitrosoguanidine) treatment.

The presence of activity of the LeuO protein can be detected by complementation of *hns⁻* mutation by the method described, for example, Shi, X. and Bennett, G.N. (J. Bacteriol., 177, 3, 810-814 (1995)). So, the reduced or absent activity of the LeuO protein in the bacterium according the present disclosure can be determined when compared to the parent unmodified bacterium.

The presence or absence of the *leuO* gene in the chromosome of a bacterium can be detected by well-known methods, including PCR, Southern blotting and the like. In addition, the level of gene expression can be estimated by measuring the amount of mRNA transcribed from the gene using various well-known methods, including Northern blotting, quantitative RT-PCR, and the like. Amount or molecular weight of the protein encoded by the gene can be measured by well-known methods, including SDS-PAGE followed by immunoblotting assay (Western blotting analysis), and the like.

Methods for preparation of plasmid DNA, digestion and ligation of DNA, transformation, selection of an oligonucleotide as a primer, and the like may be ordinary methods well known to one skilled in the art. These methods are described, for instance, in Sambrook, J., Fritsch, E.F., and Maniatis, T., "Molecular Cloning A Laboratory Manual, Second Edition", Cold Spring Harbor Laboratory Press (1989).

### L-amino acid producing bacteria

The bacterium for use in the present invention can be obtained by attenuating expression of the *leu*O gene in a bacterium which inherently has the ability to produce an L-amino acid. Alternatively, the bacterium for use in present invention can be obtained by imparting the ability to produce an L-amino acid to a bacterium already having attenuated expression of the *leuO* gene.

### L-threonine-producing bacteria

Examples of parent strains for deriving the L-threonine-producing bacteria for use in the present invention include, but are not limited to, strains belonging to the genus *Escherichia*, such as *E*. *coli* TDH-6/pVIC40 (VKPM B-3996) (U.S. Patent No. 5,175,107, U.S. Patent No. 5,705,371), *E. coli* 472T23/pYN7 (ATCC 98081) (U.S. Patent No.5,631,15'7), *E*. *coli* NRRL-21593 (U.S. Patent No. 5,939,307), *E. coli* FERM BP-3756 (U.S. Patent No. 5,474,918), *E*. *coli* FERM BP-3519 and FERM BP-3520 (U.S. Patent No. 5,376,538), *E*. *coli* MG442 (Gusyatiner et al., Genetika (in Russian), 14,947-956 (1978)), *E. coli* VL643 and VL2055 (EP 1149911 A), and the like.

The strain TDH-6 is deficient in the *thrC* gene, as well as being sucrose-assimilative, and the *ilvA* gene has a leaky mutation. This strain also has a mutation in the *rhtA* gene, which imparts resistance to high concentrations of threonine or homoserine. The strain B-3996 contains the plasmid pVIC40 which was obtained by inserting a *thrA*BC* operon which includes a mutant *thrA* gene into a RSP1010-derived vector. This mutant *thrA* gene encodes aspartokinase homoserine dehydrogenase I which has substantially desensitized feedback inhibition by threonine. The strain B-3996 was deposited on November 19,1987 in the All-Union Scientific Center of Antibiotics (Nagatinskaya Street 3-A, 117105 Moscow, Russian Federation) under the accession number RIA 1867. The strain was also deposited in the Russian National Collection of Industrial Microorganisms (VKPM) (Russia, 117545 Moscow 1, Dorozhny proezd. 1) on April 7,1987 under the accession number B-3996. *E. coli* VKPM B-5318 (EP 0593792B) may also be used as a parent strain for deriving L-threonine-producing bacteria for use in the present invention. The strain B-5318 is prototrophic with regard to isoleucine, and a temperature-sensitive lambda-phage C1 repressor and PR promoter replaces the regulatory region of the threonine operon in plasmid pVIC40. The strain VKPM B-5318 was deposited in the Russian National Collection of Industrial Microorganisms (VKPM) on May 3,1990 under accession number of VKPM B-5318.

Preferably, the bacterium for use in the present invention is additionally modified to enhance expression of one or more of the following genes:
- the mutant *thrA* gene which codes for aspartokinase homoserine dehydrogenase I resistant to feed back inhibition by threonine;
- the *thrB* gene which codes for homoserine kinase;
- the *thrC* gene which codes for threonine synthase;
- the *rhtA* gene which codes for a putative transmembrane protein;
- the *asd* gene which codes for aspartate-β-semialdehyde dehydrogenase; and
- the *aspC* gene which codes for aspartate aminotransferase (aspartate transaminase);

The *thrA* gene which encodes aspartokinase homoserine dehydrogenase I of *Escherichia coli* has been elucidated (nucleotide positions 337 to 2799, GenBank accession NC_000913.2, gi: 49175990). The *thrA* gene is located between the *thrL* and *thrB* genes on the chromosome *of E. coli* K-12. The *thrB* gene which encodes homoserine kinase of *Escherichia coli* has been elucidated (nucleotide positions 2801 to 3733, GenBank accession NC_000913.2, gi: 49175990). The *thrB* gene is located between the *thrA* and *thrC* genes on the chromosome of *E*.*coli* K-12. The *thrC* gene which encodes threonine synthase of *Escherichia coli* has been elucidated (nucleotide positions 3734 to 5020, GenBank accession NC_000913.2, gi: 49175990). The *thrC* gene is located between the *thrB* gene and the *yaaX* open reading frame on the chromosome *of E. coli* K-12. All three genes functions as a single threonine operon. To enhance expression of the threonine operon, the attenuator region which affects the transcription is desirably removed from the operon (WO2005/049808, WO2003/097839).

A mutant *thrA* gene which codes for aspartokinase homoserine dehydrogenase I resistant to feed back inhibition by threonine, as well as, the *thrB* and *thrC* genes can be obtained as one operon from well-known plasmid pVIC40 which is presented in the threonine producing *E. coli* strain VKPM B-3996. Plasmid pVIC40 is described in detail in U.S. Patent No. 5,705,371.

The *rhtA* gene exists at 18 min on the E. coli chromosome close to the *glnHPQ* operon, which encodes components of the glutamine transport system. The *rhtA* gene is identical to ORF1 (*ybiF* gene, nucleotide positions 764 to 1651, GenBank accession number AAA218541, gi:440181) and located between the *pexB* and *ompX* genes. The unit expressing a protein encoded by the ORF1 has been designated the *rhtA* gene (rht: resistance to homoserine and threonine). Also, it was revealed that the rhtA23 mutation is an A-for-G substitution at position -1 with respect to the ATG start codon (ABSTRACTS of the 17th International Congress of Biochemistry and Molecular Biology in conjugation with Annual Meeting of the American Society for Biochemistry and Molecular Biology, San Francisco, California August 24-29, 1997, abstract No. 457, EP 1013765 A).

The *asd* gene *of E. coli* has already been elucidated (nucleotide positions 3572511 to 3571408, GenBank accession NC_000913.1, gi:16131307), and can be obtained by PCR (polymerase chain reaction; refer to White, T.J. et al., Trends Genet., 5, 185 (1989)) utilizing primers prepared based on the nucleotide sequence of the gene. The *asd* genes of other microorganisms can be obtained in a similar manner.

Also, the *aspC* gene *of E. coli* has already been elucidated (nucleotide positions 983742 to 984932, GenBank accession NC_000913.1, gi:16128895), and can be obtained by PCR. The *aspC* genes of other microorganisms can be obtained in a similar manner.

### L-lysine-producing bacteria

Examples of L-lysine-producing bacteria belonging to the genus *Escherichia* include mutants having resistance to an L-lysine analogue. The L-lysine analogue inhibits growth of bacteria belonging to the genus *Escherichia,* but this inhibition is fully or partially desensitized when L-lysine coexists in a medium. Examples of the L-lysine analogue include, but are not limited to, oxalysine, lysine hydroxamate, S-(2-aminoethyl)-L-cysteine (AEC), γ-methyllysine, α-chlorocaprolactam and so forth. Mutants having resistance to these lysine analogues can be obtained by subjecting bacteria belonging to the genus *Escherichia* to a conventional Artificial mutagenesis treatment Specific examples of bacterial strains useful for producing L-lysine include *Escherichia coli* AJ11442 (FERM BP-1543, NRRL B-12185; see U.S. Patent No. 4,346,170) and *Escherichia coli* VL611. In these microorganisms, feedback inhibition of aspartokinase by L-lysine is desensitized.

The strain WC196 may be used as an L-lysine producing bacterium of *Escherichia coli.* This bacterial strain was bred by conferring AEC resistance to the strain W3110, which was derived from *Escherichia coli* K-12. The resulting strain was designated *Escherichia coli* AJ13069 strain and was deposited at the National Institute of Bioscience and Human-Technology, Agency of Industrial Science and Technology (currently National Institute of Advanced Industrial Science and Technology, International Patent Organism Depositary, Tsukuba Central 6, 1-1, Higashi 1-Chome, Tsukuba-shi, Ibaraki-ken, 305-8566, Japan) on December 6,1994 and received an accession number of FERM P-14690. Then, it was converted to an international deposit under the provisions of the Budapest Treaty on September 29,1995, and received an accession number of FERM BP-5252 (U.S. Patent No. 5,827,698).

Examples of parent strains for deriving L-lysine-producing bacteria for use in the present invention also include strains in which expression of one or more genes encoding an L-lysine biosynthetic enzyme are enhanced. Examples of such genes include, but are not limited to, genes encoding dihydrodipicolinate synthase (*dapA*)*,* aspartokinase (*lysC*), dihydrodipicolinate reductase (*dapB*)*,* diaminopimelate decarboxylase (*lysA*)*,* diaminopimelate dehydrogenase (*ddh*) (U.S. Patent No. 6,040,160), phosphoenolpyrvate carboxylase (*ppc*)*,* aspartate semialdehyde dehydrogenease (*asd*), and aspartase (*aspA*) (EP 1253195 A). In addition, the parent strains may have an increased level of expression of the gene involved in energy efficiency (*cyo*) (EP 1170376 A), the gene encoding nicotinamide nucleotide transhydrogenase (*pntAB*) (U.S. Patent No. 5,830,716), the *ybjE* gene (WO2005/073390), or combinations thereof.

Examples of parent strains for deriving L-lysine-producing bacterial for use in the present invention also include strains having decreased or eliminated activity of an enzyme that catalyzes a reaction for generating a compound other than L-lysine by branching off from the biosynthetic pathway of L-lysine. Examples of the enzymes that catalyze a reaction for generating a compound other than L-lysine by branching off from the biosynthetic pathway of L-lysine include homoserine dehydrogenase, lysine decarboxylase (U.S. Patent No. 5,827,698), and the malic enzyme (WO2005/410175).

### L-cysteine-producing bacteria

Examples of parent strains for deriving L-cysteine-producing bacteria include, but are not limited to, strains belonging to the genus *Escherichia,* such as *E. coli* JM15 which is transformed with different *cysE* alleles coding for feedback-resistant serine acetyltransferases (U.S. Patent No. 6,218,168, Russian patent application 2003121601); *E*. *coli* W3110 having over-expressed genes which encode proteins suitable for secreting substances toxic for cells (U.S. Patent No. 5,972,663); *E. coli* strains having lowered cysteine desulfohydrase activity (JP11155571A2); *E coli* W3110 with increased activity of a positive transcriptional regulator for cysteine regulon encoded by the *cysB* gene (WO0127307A1), and the like.

### L-leucine-producing bacteria

Examples of parent strains for deriving L-leucine-producing bacteria include, but are not limited to, strains belonging to the genus *Escherichia,* such as *E coli* strains resistant to leucine (for example, the strain 57 (VKPM B-7386, U.S. Patent No. 6,124,121)) or leucine analogs including p-2-thienylalanine, 3-hydroxyleucine, 4-azaleucine, 5,5,5-trifluoroleucine (JP 62-34397 B and JP 8-70879 A); *E. coli* strains obtained by the gene engineering method described in WO96/06926; *E. coli* H-9068 (JP 8-70879 A), and the like.

The bacterium may be improved by enhancing the expression of one or more genes involved in L-leucine biosynthesis. Examples include genes of the *leuABCD* operon, which are preferably represented by a mutant *leuA* gene coding for isopropylmalate synthase freed from feedback inhibition by L-leucine (US Patent 6,403,342). In addition, the bacterium may be improved by enhancing the expression of one or more genes coding for proteins which excrete L-amino acid from the bacterial cell. Examples of such genes include the b2682 and b2683 genes (*ygaZH* genes) (EP 1239041 A2).

### L-histidine-producing bacteria

Examples of parent strains for deriving L-histidine-producing bacteria include, but are not limited to, strains belonging to the genus *Escherichia,* such as *E. coli* strain 24 (VKPM B-5945, RU2003677); *E. coli* strain 80 (VKPM B-7270, RU2119536); *E. coli* NRRL B-12116 - B12121 (U.S. Patent No. 4,388,405); *E. coli* H-9342 (FERM BP-6675) and H-9343 (FERM BP-6676) (U.S. Patent No. 6,344,347); *E. coli* H-9341 (FERM BP-6674) (EP1085087); *E. coli* A180/pFM201 (U,S. Patent No. 6,258,554) and the like.

Examples of parent strains for deriving L-histidine-producing bacteria also include strains in which expression of one or more genes encoding an L-histidine biosynthetic enzyme are enhanced. Examples of such genes include genes encoding ATP phosphoribosyltransferase (*hisG*)*,* phosphoribosyl AMP cyclohydrolase (*hisI*), phosphoribosyl-ATP pyrophosphohydrolase (*hisIE*), phosphoribosylformimino-5-aminoimidazole carboxamide ribotide isomerase (*his*A), amidotransferase (*hisH*), histidinol phosphate aminotransferase (*hisC*), histidinol phosphatase (*hisB*), histidinal dehydrogenase (*hisD),* and so forth.

It is known that the L-histidine biosynthetic enzymes encoded by *hisG* and *hisBHAFI* are inhibited by L-histidine, and therefore an L-histidine-producing ability can also be efficiently enhanced by introducing a mutation conferring resistance to the feedback inhibition into ATP phosphoribosyltransferase (Russian Patent Nos. 2003677 and 2119536).

Specific examples of strains having an L-histidine-producing ability include *E*. *coli* FERM-P 5038 and 5048 which have been introduced with a vector carrying a DNA encoding an L-histidine-biosynthetic enzyme (JP 56-005099 A), *E*. *coli* strains introduced with rht, a gene for an amino acid-export (EP101b710A), *E. coli* 80 strain imparted with sulfaguanidine, DL-1,2,4-triazole-3-alanine, and streptomycin-resistance (VKPM B-7270, Russian Patent No. 2119536), and so forth.

### L-glutamic acid-producing bacteria

Examples of parent strains for deriving L-glutamic acid-producing bacteria include, but are not limited to, strains belonging to the genus *Escherichia,* such as *E. coli* VL334thrC⁺ (EP 1172433). *E. coli* VL334 (VKPM B-1641) is an L-isoleucine and L-threonine auxotrophic strain having mutations in *thrC* and *ilvA* genes (U.S. Patent No. 4,278,765). A wild-type allele of the *thrC* gene was transferred by the method of general transduction using a bacteriophage P1 grown on the wild-type *E. coli* strain K12 (VKPM B-7) cells. As a result, an L-isoleucine auxotrophic strain VL334thrC⁺ (VKPM B-8961), which is able to produce L-glutamic acid, was obtained.

Examples of parent strains for deriving the L-glutamic acid-producing bacteria include, but are not limited to, strains in which expression of one or more genes encoding an L-glutamic acid biosynthetic enzyme are enhanced. Examples of such genes include genes encoding glutamate dehydrogenase (*gdhA*), glutamine synthetase (*glnA*), glutamate synthetase (*gltAB*), isocitrate dehydrogenase (*icdA*), aconitate hydratase (*acnA*, *acnB*)*,* citrate synthase (*gltA*), phosphoenolpyruvate carboxylase (*ppc*), pyruvate carboxylase (*pyc*)*,* pyruvate dehydrogenase (*aceEF, IpdA*)*,* pyruvate kinase (*pykA,pykF*)*,* phosphoenolpyruvate synthase (*ppsA*), enolase (*eno*)*,* phosphoglyceromutase (*pgmA, pgmI*)*,* phosphoglycerate kinase (*pgk*), glyceraldehyde-3-phophate dehydrogenase (*gapA*), triose phosphate isomerase (*tpiA*), fructose bisphosphate aldolase (*fbp*), phosphofructokinase (*pfkA*, *pfkB*), and glucose phosphate isomerase (*pgi*).

Examples of strains modified so that expression of the citrate synthetase gene, the phosphoenolpyruvate carboxylase gene, and/or the glutamate dehydrogenase gene is/are enhanced include those disclosed in EF1078989A, EP955368A, and EP952221A.

Examples of parent strains for deriving the L-glutamic acid-producing bacteria also include strains having decreased or eliminated activity of an enzyme that catalyzes synthesis of a compound other than L-glutamic acid by branching off from an L-glutamic acid biosynthesis pathway. Examples of such enzymes include isocitrate lyasc (*aceA*)*,* α-ketoglutarate dehydrogenase (*sucA*), phosphotransacetylase (*pta*), acetate kinase (*ack*), acetohydroxy acid synthase (*ilvG*), acetolactate synthase (*ilvI*)*,* formate acetyltransferase (*pfl*), lactate dehydrogenase (*ldh*)*,* and glutamate decarboxylase (*gadAB*)*.* Bacteria belonging to the genus *Escherichia* deficient in the α-ketoglutarate dehydrogenase activity or having a reduced α-ketoglutarate dehydrogenase activity and methods for obtaining them are described in U.S. Patent Nos. 5,378,616 and 5,573,945. Specifically, these strains include the following:
*E. coli* W3110sucA::Kmr
*E. coli* AJ12624 (FERM BP-3853)
E. *coli* AJ12628 (FERM BP-3854)
*E. coli* A12949 (FERM BP-4881)

*E. coli* W3110sucA::Kmr is a strain obtained by disrupting the α-ketoglutarate dehydrogenase gene (hereinafter referred to as *"sucA* gene") of *E*. *coli* W3110. This strain is completely deficient in the α-ketoglutarate dehydrogenase.

Other examples of L-glutamic acid-producing bacterium include those which belong to the genus Escherichia and have resistance to an aspartic acid antimetabolite. These strains can also be deficient in the α-ketoglutarate dehydrogenase activity and include, for example, *E*. *coli* AJ13199 (FERM BP-5807) (U.S. Patent No. 5.908,768), FFRM P-12379, which additionally has a low L-glutamic acid decomposing ability (U.S. Patent No. 5,393,671); AJ13138 (FERM BP-5565) (U.S. Patent No. 6,110,714), and the like.

Examples of L-glutamic acid-producing bacteria, include mutant strains belonging to the genus *Pantoea* which are deficient in the α-ketoglutarate dehydrogenase activity or have a decreased α-ketoglutarate dehydrogenase activity, and can be obtained as described above. Such strains include *Pantoea ananatis* AJ13356. (U.S. Patent No. 6,331,419). *Pantoea ananatis* AJ13356 was deposited at the National Institute of Bioscience and Human-Technology, Agency of Industrial Science and Technology, Ministry of International Trade and Industry (currently, National Institute of Advanced Industrial Science and Technology, International Patent Organism Depositary, Central 6, 1-1, Higashi 1-Chome, Tsukuba-shi, Ibaraki-ken, 305-8566, Japan) on February 19, 1998 under an accession number of FERM P-16645. It was then converted to an international deposit under the provisions of Budapest Treaty on January 11,1999 and received an accession number of FERM BP-6615. *Pantoea ananatis* AJ13356 is deficient in the α-ketoglutarate dehydrogenase activity as a result of disruption of the αKGDH-E1 subunit gene (*sucA*)*.* The above strain was identified as *Enterobacter agglomerans* when it was isolated and deposited as the *Enterobacter agglomerans* AJ13356. However, it was recently re-classified as *Pantoea ananatis* on the basis of nucleotide sequencing of 16S rRNA and so forth. Although AJ13356 was deposited at the aforementioned depository as *Enterobacter agglomerans,* for the purposes of this specification, they are described as *Pantoea ananatis.*

### L-phenylolanine-producing bacteria

Examples of parent strains for deriving L-phenylalaninc-producing bacteria include, but are not limited to, strains belonging to the genus *Escherichia,* such as *E. coli* AJ12739 (tyrA::Tn10, tyrR) (VKPM B-8197); *E. coli* HW1089 (ATCC 55371) harboring the mutant pheA34 gene (U.S. Patent No. 5,354,672); *E. coli* MWEC101-b (KR8903681); *E. coli* NRRL B-12141, NRRL B-12145, NRRL B-12146 and NRRL B-12147 (U.S. Patent No. 4,407,952). Also, as a parent strain, *E*. *coli* K-12 [W3110 (tyrA)/pPHAB (FERM *BP-3566), E*. *coli* K-12 [W3110 (tyrA)/pPHAD] (FERM BP-12659), *E. coli* K-12 [W3110 (tyrA)/pPRATerm] (FERM BP-12662) and *coli* K-12 [W3110 (tyrA)/pBR-aroG4, pACMAB] named as AJ 12604 (FERM BP-3579) may be used (EP 488424 B1). Furthermore, L-phenylalanine producing bacteria belonging to the genus *Escherichia* with an enhanced activity of the protein encoded by the *yed*A gene or the *yddG* gene may also be used (U.S. patent applications 200310148473 A1 and 2003/0157667 A1).

### L-tryptophan-producing bacteria

Examples of parent strains for deriving the L-tryptophan-producing bacteria include, but are not limited to, strains belonging to the genus *Escherichia,* such as *E*. *coli* JP4735/pMU3028 (DSM10122) and JP6015/pMU91 (DSM10123) deficient in the tryptophanyl-tRNA synthetase encoded by mutant *trpS* gene (U.S. Patent No. 5,756,345); *E*. *coli* SV164 (pGH5) having a *serA* allele encoding phosphoglycerate dehydrogenase free from feedback inhibition by serine and a *trpE* allele encoding anthranilate synthase free from feedback inhibition by tryptophan (U.S. Patent No. 6,180,373); *E. coli* AGX17 (pGX44) (NRRL B-12263) and AGX6(pGX50)aroP (NRRL B-12264) deficient in the enzyme tryptophanase (U.S. Patent No. 4,371,614); *E. coli* AGX17/pGX50,pACKG4-pps in which a phosphoenolpyruvate-producing ability is enhanced (WO9708333, U.S. Patent No. 6,319,696), and the like may be used. L-tryptophan-producing bacteria belonging to the genus *Escherichia* with an enhanced activity of the protein encoded by the *yedA* gene or the *yddG* gene may also be used (U.S. patent applications 2003/0148473 A1 and 2003/0157667 A1).

Examples of parent strains for deriving the L-tryptophan-producing bacteria also include strains in which one or more activities of the enzymes selected from anthranilate synthase, phosphoglycerate dehydrogenase, and tryptophan synthase are enhanced. The anthranilate synthase and phosphoglycerate dehydrogenase are both subject to feedback inhibition by L-tryptophan and L-serine, so that a mutation desensitizing the feedback inhibition may be introduced into these enzymes. Specific examples of strains having such a mutation include a *E*. *coli* SV164 which harbors desensitized anthranilate synthase and a transformant strain obtained by introducing into the *E. coli* SV164 the plasmid pGH5 (WO 94/08031), which contains a mutant serA gene encoding feedback-desensitized phosphoglycerate dehydrogenase.

Examples of parent strains for deriving the L-tryptophan-producing bacteria also include strains into which the tryptophan operon which contains a gene encoding desensitized anthranilate synthase has been introduced (JP 57-71397 A, JP 62-244382 A, U.S. Patent No. 4,371,614), Moreover, L-tryptophan-producing ability may be imparted by enhancing expression of a gene which encodes tryptophan synthase, among tryptophan operons (*trpBA*)*.* The tryptophan synthase consists of α and β subunits which are encoded by the *trpA* and *trpB* genes, respectively. In addition, L-tryptophan-producing ability may be improved by enhancing expression of the isocitrate lyase-malate synthase operon (WO2005/103275).

### L-proline-producing bacteria

Examples of parent strains for deriving L-proline-producing bacteria include, but are not limited to, strains belonging to the genus *Escherichia,* such as *E. coli* 702ilvA (VKPM B-8012) which is deficient in the *ilvA* gene and is able to produce L-proline (EP 1172433). The bacterium may be improved by enhancing the expression of one or more genes involved in L-proline biosynthesis. Examples of such genes for L-proline producing bacteria which are preferred include the *proB* gene coding for glutamate kinase of which feedback inhibition by L-proline is desensitized (DE Patent 3127361). In addition, the bacterium may be improved by enhancing the expression of one or more genes coding for proteins excreting L-amino acid from bacterial cell. Such genes are exemplified by b2682 and b2683 genes (*ygaZH* genes) (EP1239041 A2).

Examples of bacteria belonging to the genus *Escherichia,* which have an activity to produce L-proline include the following *E. coli* strains: NRRL B-12403 and NRRL B-12404 (GB Patent 2075056), VKPM B-8012 (Russian patent application 2000124295), plasmid mutants described in DE Patent 3127361, plasmid mutants described by Bloom F.R. et al (The 15th Miami winter symposium, 1983, p.34), and the like.

### L-arginine-producing bacteria

Examples of parent strains for deriving L-arginine-producing bacteria for use in the present invention include, but arc not limited to, strains belonging to the genus *Escherichia,* such as *E. coli* strain 237 (VKPM B-7925) (U.S. Patent Application 2002/058315 Al) and its derivative strains harboring mutant N-acetylglutamate synthase (Russian Patent Application No. 2001112869), *E. coli* strain 382 (VKPM B-7926) (EP1170358A1), an arginine-producing strain into which *argA* gene encoding N-acetylglutamate synthetase is introduced therein (EP1170361A1), and the like.

Examples of parent strains for deriving L-arginine producing bacteria for use in the present invention also include strains in which expression of one or more genes encoding an L-arginine biosynthetic enzyme are enhanced. Examples of such genes include genes encoding N-acetylglutamyl phosphate reductase (*argC*)*,* ornithine acetyl transferase (*argJ*)*,* N-acetylglutamate kinase (*argB*)*,* acetylornithine transaminase (*argD*)*,* ornithine carbamoyl transferase (*argF*)*,* argininosuccinic acid synthetase (*argG*)*,* argininosuccinic acid lyase (*argH*)*,* and carbamoyl phosphate synthetase (*carAB*)*.*

### L-valine-producing bacteria

Example of parent strains for deriving L-valine-producing bacteria include, but are not limited to, strains which have been modified to overexpress the *ilvGMEDA* operon (U.S. Patent No. 5,998,178). It is desirable to remove the region of the *ilvGMEDA* operon which is required for attenuation so that expression of the opemn is not attenuated by L-valine that is produced. Furthermore, the *ilva* gene in the operon is desirably disrupted so that threonine deaminase activity is decreased.

Examples of parent strains for deriving L-valine-producing bacteria also include mutants having a mutation of amino-acyl t-RNA synthetase (U.S. Patent No. 5,658,766). For example, *E. coli* VL1970, which has a mutation in the *ileS* gene encoding isoleucine tRNA synthetase, can be used. *E. coli* VL1970 has been deposited in the Russian National Collection of Industrial Microorganisms (VKPM) (Russia, 113545 Moscow, 1 Dorozhny Proezd, 1) on June 24, 1988 under accession number VKPM B-4411.

Furthermore, mutants requiring lipoic acid for growth and/or lacking H⁺-ATPase can also be used as parent strains (WO96/06926).

### L-isoleucine-producing bacteria

Examples of parent strains for deriving L-isoleucine producing bacteria include, but are not limited to, mutants having resistance to 6-dimethylaminopurine (JP 5-304969 A), mutants having resistance to an isoleucine analogue such as thiaisoleucine and isoleucine hydroxamate, and mutants additionally having resistance to DL-ethionine and/or arginine hydroxamate (JP 5-130882 A). In addition, recombinant strains transformed with genes encoding proteins involved in L-isoleucine biosynthesis, such as threonine deaminase and acetohydroxate synthase, can also be used as parent strains (JP 2-458 A, FR 0356739, and U.S. Patent No. 5,998,178).

### 2. Method of the present invention

The method of the present invention is a method for producing an L-amino acid comprising cultivating the bacterium for use in the present invention in a culture medium to produce and excrete the L-amino acid into the medium, and collecting the L-amino acid from the medium, wherein said L-amino acid is selected from the group consisting of L-threonine, L-lysine, L-methionine, L-asparagine, L-aspartic acid, and L-arginine.

In the present invention, the cultivation, collection, and purification of an L-amino acid from the medium and the like may be performed in a manner similar to conventional fermentation methods wherein an amino acid is produced using a bacterium.

A medium used for culture may be either a synthetic or natural medium, so long as the medium includes a carbon source and a nitrogen source and minerals and, if necessary, appropriate amounts of nutrients which the bacterium requires for growth. The carbon source may include various carbohydrates such as glucose and sucrose, and various organic acids. Depending on the mode of assimilation of the used microorganism, alcohol, including ethanol and glycerol, may be used. As the nitrogen source, various ammonium salts such as ammonia and ammonium sulfate, other nitrogen compounds such as amines, a natural nitrogen source such as peptone, soybean-hydrolysate, and digested fermentative microorganism can be used. As minerals, potassium monophosphate, magnesium sulfate, sodium chloride, ferrous sulfate, manganese sulfate, calcium chloride, and the like can be used. As vitamins, thiamine, yeast extract, and the like, can be used.

The cultivation is preferably performed under aerobic conditions, such as a shaking culture, and a stirring culture with aeration, at a temperature of 20 to 40 °C, preferably 30 to 38 °C. The pH of the culture is usually between 5 and 9, preferably between 6.5 and 7.2. The pH of the culture can be adjusted with ammonia, calcium carbonate, various acids, various bases, and buffers. Usually, a 1 to 5-day cultivation leads to accumulation of the target L-amino acid in the liquid medium.

After cultivation, solids such as cells can be removed from the liquid medium by centrifugation or membrane filtration, and then the Amino acid can be collected and purified by ion-exchange, concentration, and/or crystallization methods.

### Brief Description of Drawings

Figure 1 shows the relative positions of primers *leuO* L and *leuO* R on plasmid pACYC184, which is used for amplification of the *cat* gene.
Figure 2 shows the construction of the chromosomal DNA fragment containing the inactivated *leuO* gene.

### Examples

The present invention will be more concretely explained below.

### Example 1. Construction of a strain with an inactivated leuO gene.

### 1. Deletion of the leuO genre.

A strain having deletion of the *leuO* gene was constructed by the method initially developed by Datsenko, K.A. and Wanner, B.L. (Proc. Natl. Acad. Sci. USA, 2000, 97(12), 6640-6645) called "Red-driven integration". According to this procedure, the PCR primers *leuO* L (SEQ ID NO: 3) and *leuO* R (SEQ ID NO: 4), which are homologous to both the regions adjacent to the *leuO* gene and the gene conferring antibiotic resistance, respectively, in the template plasmid, were constructed. The plasmid pACYC184 (NBL Gene Sciences Ltd., UK) (GenBank/EMBL accession number X06403) was used as a template in the PCR reaction. Conditions for PCR were as follows: denaturation step: 3 min at 95°C; profile for two first cycles: 1 min at 95°C, 30 sec at 50°C, 40 sec at 72 °C; profile for the last 25 cycles: 30 sec at 95 °C, 30 sec at 54°C, 40 sec at 72 °C; final step: 5 min at 72 °C.

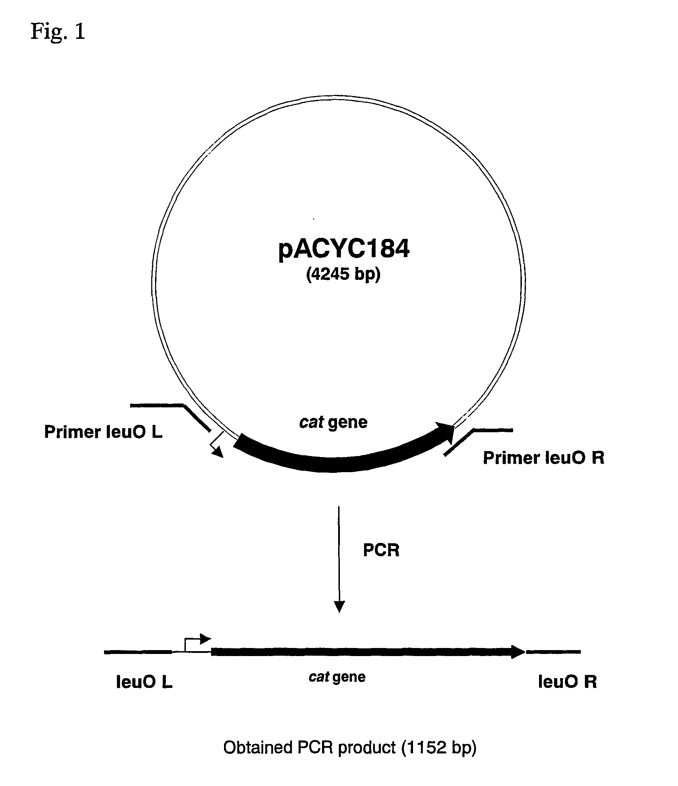

A 1152 bp PCR product (Fig. 1) was obtained and purified in agarose gel and was used for electroporation of *E. coli* MG1655 (ATCC 700926), which contains the plasmid pKD46 having a temperature-sensitive replication. The plasmid pKD46 (Datsenko, K.A. and Wanner, B.L., Proc. Natl. Acad. Sci. USA, 2000, 97:12:6640-45) includes a 2,154 nucleotide (31088-33241) DNA fragment of phage λ (GenBank accession No. J02459), and contains genes of the λ Red homologous recombination system (γ, β, exo genes) under the control of the arabinose-inducible P_{araB} promoter. The plasmid pKD46 is necessary for integration of the PCR product into the chromosome of strain MG1655.

Electrocompetent cells were prepared as follows: *E. coli* MG1655/pKD46 was grown overnight at 30°C in LB medium containing ampicillin (100 mg/l), and the culture was diluted 100 times with 5 ml of SOB medium (Sambrook et al, "Molecular Cloning A Laboratory Manual, Second Edition", Cold Spring Harbor Laboratory Press (1989)) containing ampicillin and L-arabinose (1 mM). The cells were grown with aeration at 30°C to an OD₆₀₀ of ≈0.6 and then were made electrocompetent by concentrating 100-fold and washing three times with ice-cold deionized H₂O. Electroporation was performed using 70 µl of cells and ≈100 ng of PCR product. Cells after electroporation were incubated with 1 ml of SOC medium (Sambrook et al, "Molecular Cloning A Laboratory Manual, Second Edition", Cold Spring Harbor Laboratory Press (1989)) at 37°C for 2.5 hours and then were plated onto L-agar containing chloramphenicol (30 µg/ml) and grown at 37 °C to select Cm^{R} recombinants. Then, to eliminate the pKD46 plasmid, 2 passages on L-agar with Cm at 42°C were performed and the obtained colonies were tested for sensitivity to ampicillin.

### 2. Verification of the leuO gene deletion by PCR.

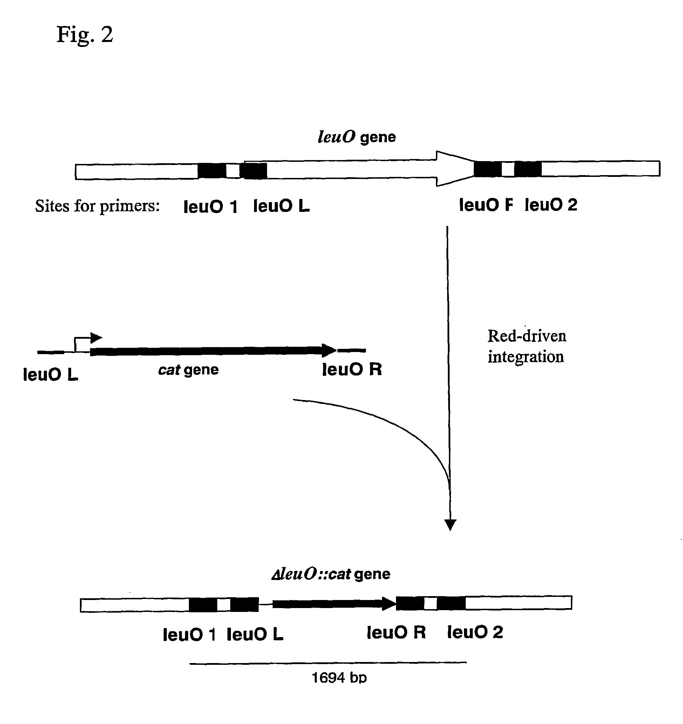

The mutants, which have the *leuO* gene deleted, and marked with the Cm resistance gene, were verified by PCR. Locus-specific primers *leuO* 1 (SEQ ID NO: 5) and *leuO* 2 (SEQ ID NO: 6) were used in PCR for verification. Conditions for PCR verification were as follows: denaturation step for 3 min at 94°C; profile for the 30 cycles: 30 sec at 94°C, 30 sec at 54°C, 1 min at 72 °C; final step: 7 min at 72 °C. The PCR product obtained in the reaction with the cells of the parental *leuO* ⁺ strain MG1655 as the template was 1121 bp in length. The PCR product obtained in the reaction with the cells of the mutant strain as the template was 1694 bp in length (Fig.2). The mutant strain was named MG1655 *ΔleuO:: cat.*

### Example 2. Production of L-threonine by E. coli B-3996-ΔleuO.

To test the effect of inactivation of the *leuO* gene on threonine production, DNA fragments from the chromosome of the above-described *E. coli* MG1655 *ΔleuO::cat* were transferred to the threonine-producing *E. coli* strain VKPM B-3996 by P1 transduction (Miller, J.H. (1972) Experiments in Molecular Genetics, Cold Spring Harbor Lab. Press, Plainview, NY) to obtain the strain B-3996-Δ*leuO*.

Both *E. coli* B-3996 and B-3996-Δ*leuO* were grown for 18-24 hours at 37°C on L-agar plates. To obtain a seed culture, the strains were grown on a rotary shaker (250 rpm) at 32°C for 18 hours in 20x200 mm test tubes containing 2 ml of L-broth with 4% sucrose. Then, the fermentation medium was inoculated with 0.21 ml (10%) seed material. The fermentation was performed in 2 ml of minimal medium for fermentation in 20x200 mm test tubes. Cells were grown for 72 hours at 32°C with shaking at 250 rpm.

After cultivation, the amount of L-threonine which had accumulated in the medium was determined by paper chromatography using the following mobile phase: butanol : acetic acid : water = 4:1: 1 (v/v). A solution (2%) of ninhydrin in acetone was used as a visualizing reagent. A spot containing L-threonine was cut out, L-threonine was eluted in 0.5 % water solution of CdCl₂, and the amount of L-threonine was estimated spectrophotometrically at 540 nm. The results of 8 independent test tube fermentations are shown in Table 1.

The composition of the fermentation medium (g/l) was as follows:

| | |
|---|---|
| Glucose | 80.0 |
| (NH₄)₂SO₄ | 22.0 |
| NaCl | 0.8 |
| KH₂PO₄ | 2.0 |
| MgSO₄·7H₂O | 0.8 |
| FeSO₄·7H₂O | 0.02 |
| MnSO₄·5H₂O | 0.02 |
| Thiamine HCl | 0.0002 |
| Yeast extract | 1.0 |
| CaCO₃ | 30.0 |

Glucose and magnesium sulfate were sterilized separately. CaCO₃ was sterilized by dry-heat at 180°C for 2 hours. The pH was adjusted to 7.0. Antibiotic was introduced into the medium after sterilization.

**Table 1**

| Strain | OD₅₄₀ | Amount of L-threonine, g/l |
|---|---|---|
| B-3996 | 25.2 ± 2.6 | 28.4 ± 0.7 |
| B-3996-Δ*leuO* | 23.3 ± 0.5 | 32.7 ± 1.0 |

It can be seen from the Table 1, B-3996-Δ*leuO* caused a higher amount of accumulation of L-threonine as compared with B-3996.

### Example 3. Production of L-lysine by E. coli WC196-ΔleuO.

To test the effect of inactivation of the *leuO* gene on lysine production, DNA fragments from the chromosome of the above-described *E. coli* MG1655 *ΔleuO::cat* were transferred to the lysine-producing *E. coli* strain WC196 (FERM BP-5252) by P1 transduction (Miller, J.H. (1972) Experiments in Molecular Genetics, Cold Spring Harbor Lab. Press, Plainview, NY) to obtain the strain WC196-Δ*leuO*.

To obtain a seed culture, both *E. coli* WC196 and WC196-Δ*leuO* were grown on a rotary shaker (250 rpm) at 32 °C for 18 hours in 20x200-mm test tubes containing 2 ml of medium diluted two times compared to the fermentation medium described below. Then 0.21 ml (10%) of the seed culture was inoculated into 2 ml of the fermentation medium in 20x200 mm test tubes. The fermentation was performed at 32 °C for 24 hours with shaking at 250 rpm.

After cultivation, the amount of L-lysine which had accumulated in the medium was determined by paper chromatography using the following mobile phase: butanol - acetic acid - water = 4 : 1 : 1 (v/v). A solution of ninhydrin (2%) in acetone was used as a visualizing reagent. A spot containing L-lysine was cut out, L-lysine was eluted with 0.5% water solution of CdCl₂, and the amount of L-lysine was estimated spectrophotometrically at 540 nm. The results of five independent test-tube fermentations are shown in Table 2.

The composition of the fermentation medium (g/l) was as follows:

| | |
|---|---|
| Glucose | 40.0 |
| (NH₄)₂SO₄ | 24.0 |
| KH₂PO₄ | 1.0 |
| MgSO₄•7H₂O | 1.0 |
| FeSO₄·7H₂O | 0.01 |
| MnSO₄·5H₂O | 0.01 |
| Yeast extract | 2.0 |
| CaCO₃ | 30.0 |

Glucose, potassium phosphate and magnesium sulfate were sterilized separately. CaCO₃ was sterilized by dry-heat at 180°C for 2 hours. The pH was adjusted to 7.0.

**Table 2**

| Strain | OD₅₄₀ | Amount of L-lysine, g/l |
|---|---|---|
| WC196 | 26.2 ± 0.5 | 2.1 ± 0.1 |
| WC196-Δ*leuO* | 26.9 ± 0.3 | 2.4 ± 0.1 |

As follows from Table 2, WC196-Δ*leuO* caused accumulation of a higher amount of L-lysine, as compared with WC196.

### Example 4. Production of L-cysteine by E. coli JM15(ydeD)-ΔleuO.

To test the effect of inactivation of the *leuO* gene on L-cysteine production, DNA fragments from the chromosome of the above-described *E. coli* MG1655 Δ*leuO*::cat can be transferred to the *E. coli* L-cysteine producing strain JM15(ydeD) by P1 transduction (Miller, J.H. (1972) Experiments in Molecular Genetics, Cold Spring Harbor Lab. Press, Plainview, NY) to obtain the strain JM15(ydeD)-Δ*leuO*.

*E. coli* JM15(ydeD) is a derivative of *E. coli* JM15 (U.S. Patent No. 6,218,168) which can be transformed with DNA having the *ydeD* gene, which codes for a membrane protein, and is not involved in a biosynthetic pathway of any L-amino acid (U.S. Patent No. 5,972,663). The strain JM15 (CGSC# 5042) can be obtained from The Coli Genetic Stock Collection at the *E. coli* Genetic Resource Center, MCD Biology Department, Yale University (http://cgsc.biology.yale.edu/).

Fermentation conditions for evaluation of L-cysteine production are described in detail in Example 6 of U.S. Patent No. 6,218,168.

### Example 5. Production of L-leucine by E. coli 57-ΔleuO.

To test the effect of inactivation of the *leuO* gene on L-leucine production, DNA fragments from the chromosome of the above-described *E. coli* MG1655 Δ*leuO::cat* can be transferred to the *E. coli* L-leucine producing strain 57 (VKPM B-7386, U.S. Patent No. 6,124,121) by P1 transduction (Miller, J.H. (1972) Experiments in Molecular Genetics, Cold Spring Harbor Lab. Press, Plainview, NY) to obtain the strain 57-Δ*leuO*. The strain 57 has been deposited in the Russian National Collection of Industrial Microorganisms (VKPM) (Russia, 117545 Moscow, 1 Dorozhny proezd, 1) on May 19, 1997_under accession number VKPM B-7386.

Both *E. coli* 57 and 57-Δ*leuO* can be cultured for 18-24 hours at 37°C on L-agar plates. To obtain a seed culture, the strains can be grown on a rotary shaker (250 rpm) at 32 °C for 18 hours in 20x200 mm test tubes containing 2 ml of L-broth with 4% sucrose. Then, the fermentation medium can be inoculated with 0.21 ml (10%) seed material. The fermentation can be performed in 2 ml of minimal medium for fermentation in 20x200 mm test tubes. Cells can be grown for 48-72 hours at 32°C with shaking at 250 rpm. The amount of L-leucine can be measured by paper chromatography (liquid phase composition: butanol - acetic acid - water = 4:1:1)

The composition of the fermentation medium (g/l) is as follows (pH 7.2):

| | |
|---|---|
| Glucose | 60.0 |
| (NH₄)₂SO₄ | 25.0 |
| K₂HPO₄ | 2.0 |
| MgSO₄·7H₂O | 1.0 |
| Thiamine | 0.01 |
| CaCO₃ | 25.0 |

Glucose and CaCO₃ are be sterilized separately.

### Example 6. Production of L-histidine by E. coli 80-ΔleuO.

To test the effect of inactivation of the *leuO* gene on L-histidine production, DNA fragments from the chromosome of the above-described *E. coli* MG1655 Δ*leuO::cat* can be transferred to the histidine-producing *E. coli* strain 80 by P1 transduction (Miller, J.H. (1972) Experiments in Molecular Genetics, Cold Spring Harbor Lab. Press, Plainview, NY) to obtain the strain *80-*Δ*leuO.* The strain 80 has been described in Russian patent 2119536 and deposited in the Russian National Collection of Industrial Microorganisms (Russia, 117545 Moscow, 1 Dorozhny proezd, 1) on October 15, 1999 under accession number VRPM B-7270 and then converted to a deposit under the Budapest Treaty on July 12, 2004.

Both *E. coli* 80 and 80-Δ*leuO* can be cultivated in L-broth for 6 hours at 29 °C. Then, 0.1 ml of obtained cultures can each be inoculated into 2 ml of fermentation medium in 20x200mm test tube and cultivated for 65 hours at 29 °C with a rotary shaker (350 rpm). After cultivation, the amount of histidine, which accumulates in the medium, can be determined by paper chromatography. The paper can be developed with a mobile phase: n-butanol : acetic acid : water = 4 : 1 : 1 (v/v). A solution of ninhydrin (0.5%) in acetone can be used as a visualizing reagent.

The composition of the fermentation medium (g/l) is as follows (pH 6.0):

| | |
|---|---|
| Glucose | 100.0 |
| Mameno (soybean hydrolysate) | 0.2 of as total nitrogen |
| L-proline | 1.0 |
| (NH₄)₂SO₄ | 25.0 |
| KH₂PO₄ | 2.0 |
| MgSO₄•7H₂O | 1.0 |
| FeSO₄•7H₂O | 0.01 |
| MnSO₄ | 0.01 |
| Thiamine | 0.001 |
| Betaine | 2.0 |
| CaCO₃ | 60.0 |

Glucose, proline, betaine and CaCO₃ are sterilized separately. pH is adjusted to 6.0 before sterilization.

### Example 7. Production of L-glutamate by E. coli VL334thrC⁺-ΔleuO.

To test the effect of inactivation of the *leuO* gene on L-glutamate production, DNA fragments from the chromosome of the above-described *E. coli* MG1655 Δ*leuO::cat* can be transferred to the *E. coli* L-glutamate producing strain VL334thrC⁺ (EP 1172433) by P1 transduction (Miller, J.H. (1972) Experiments in Molecular Genetics, Cold Spring Harbor Lab. Press, Plainview, NY) to obtain the strain VL334thrC⁺-Δ*leuO*. The strain L334thrC⁺ has been deposited in the Russian National Collection of Industrial Microorganisms (VKPM) (Russia, 117545 Moscow, 1 Dorozhny proezd, 1) on December 6, 2004 under the accession number B-8961 and then converted to a deposit under the Budapest Treaty on December 8, 2004.

Both strains, VL334thrC⁺ and VL334thrC⁺-Δ*leuO*, can be grown for 18-24 hours at 37 °C on L-agar plates. Then, one loop of the cells can be transferred into test tubes containing 2ml of fermentation medium. The fermentation medium contains 60g/l glucose, 25 g/l ammonium sulfate, 2g/l KH₂PO₄, 1 g/l MgSO₄, 0.1 mg/ml thiamine, 70 µg/ml L-isoleucine and 25 g/l CaCO₃ (pH 7.2). Glucose and CaCO₃ are sterilized separately. Cultivation can be carried out at 30 °C for 3 days with shaking. After the cultivation, the amount of L-glutamic acid produced can be determined by paper chromatography (liquid phase composition: butanol-acetic acid-water=4:1:1) with subsequent staining by ninhydrin (1% solution in acetone) and further elution of the compounds in 50% ethanol with 0.5% CdCl₂.

### Example 8. Production of L- phenylalanine by E. coli AJ12739-ΔleuO.

To test the effect of inactivation of the *leuO* gene on L-phenylalanine production, DNA fragments from the chromosome of the above-described *E. coli* MG1655 Δ*leuO::cat* can be transferred to the phenylalanine-producing *E. coli* strain AJ12739 by P1 transduction (Miller, J.H. (1972) Experiments in Molecular Genetics, Cold Spring Harbor Lab. Press, Plainview, NY) to obtain the strain AJ12739-Δ*leuO*. The strain AJ12739 has been deposited in the Russian National Collection of Industrial Microorganisms (VKPM) (Russia, 117545 Moscow, 1 Dorozhny proezd, 1) on November 6, 2001 under accession number VKPM B-8197 and then converted to a deposit under the Budapest Treaty on August 23, 2002.

Both strains, AJ12739-Δ*leuO* and AJ12739, can be cultivated at 37 °C for 18 hours in a nutrient broth. 0.3 ml of the obtained cultures can each be inoculated into 3 ml of a fermentation medium in a 20 x 200 mm test tube and cultivated at 37 °C for 48 hours with a rotary shaker. After cultivation, the amount of phenylalanine, which accumulates in the medium can be determined by TLC. 10 x 15 cm TLC plates coated with 0.11 mm layers of Sorbfil silica gel without fluorescent indicator (Stock Company Sorbpolymer, Krasnodar, Russia) can be used. Sorbfil plates can be developed with a mobile phase: propan-2-ol : ethylacetate: 25% aqueous ammonia: water = 40 : 40 : 7 : 16 (v/v). A solution (2%) of ninhydrin in acetone can be used as a visualizing reagent.

The composition of the fermentation medium is as follows (g/l):

| | |
|---|---|
| Glucose | 40.0 |
| (NH₄)₂SO₄ | 16.0 |
| K₂HPO₄ | 0.1 |
| MgSO₄·7H₂O | 1.0 |
| FeSO₄·7H₂O | 0.01 |
| MnSO₄·5H₂O | 0.01 |
| Thiamine HCl | 0.0002 |
| Yeast extract | 2.0 |
| Tyrosine | 0.125 |
| CaCO₃ | 20.0 |

Glucose and magnesium sulfate are sterilized separately. CaCO₃ is sterilized by dry-heat at 180°C for 2 hours. pH is adjusted to 7.0.

### Example 9. Production of L- tryptophan by E. coli SV164 (pGH5)-ΔleuO.

To test the effect of inactivation of the *leuO* gene on L-tryptophan production, DNA fragments from the chromosome of the above-described *E. coli* MG1655 Δ*leuO::cat* can be transferred to the tryptophan-producing *E. coli* strain SV164 (pGH5) by P1 transduction (Miller, J.H. (1972) Experiments in Molecular Genetics, Cold Spring Harbor Lab. Press, Plainview, NY) to obtain the strain SV164(pGH5)-Δ*leuO*. The strain SV164 has the *trpE* allele encoding anthranilate synthase free from feedback inhibition by tryptophan. The plasmid pGH5 harbors a mutant *serA* gene encoding phosphoglycerate dehydrogenase free from feedback inhibition by serine. The strain SV164 (pGH5) is described in detail in U.S. Patent No. 6,180,373.

Both strains, SV164(pGH5)-Δ*leuO* and SV164(pGH5), can be cultivated with shaking at 37 °C for 18 hours in a 3 ml of nutrient broth supplemented with 20 µg/ml of tetracycline (marker of pGH5 plasmid). 0.3 ml of the obtained cultures can be inoculated into 3 ml of a fermentation medium containing tetracycline (20 µg/ml) in 20 x 200 mm test tubes, and cultivated at 37 °C for 48 hours with a rotary shaker at 250 rpm. After cultivation, the amount of tryptophan, which accumulates in the medium can be determined by TLC as described in Example 8. The fermentation medium components are set forth in Table 3, but should be sterilized in separate groups A, B, C, D, E, F, and H, as shown, to avoid adverse interactions during sterilization.

**Table 3**

| Groups | Component | Final concentration, g/l |
|---|---|---|
| A | KH₂PO₄ | 1.5 |
| | NaCl | 0.5 |
| | (NH₄)₂SO₄ | 1.5 |
| | L-Methionine | 0.05 |
| | L-Phenylalanine | 0.1 |
| | L-Tyrosine | 0.1 |
| | Mameno (total N) | 0,07 |
| B | Glucose | 40.0 |
| | MgSO₄·7H₂O | 0.3 |
| C | CaCl₂ | 0.011 |
| D | FeSO₄·7H₂O | 0.075 |
| | Sodium citrate | 1.0 |
| E | Na₂MoO₄•2H₂O | 0.00015 |
| | H₃BO₃ | 0.0025 |
| | CoCl₂·6H₂O | 0.00007 |
| | CuSO₄·5H₂O | 0.00025 |
| | MnCl₂·4H₂O | 0.0016 |
| | ZnSO₄·7 H₂O | 0.0003 |
| F | Thiamine HCl | 0.005 |
| G | CaCO₃ | 30.0 |
| H | Pyridoxine | 0.03 |

Group A has pH 7.1 adjusted by NH₄OH. Each of groups A, B, C, D, E, F and H is sterilized separately, chilled, and mixed together, and then CaCO₃ sterilized by dry heat is added to the complete fermentation medium.

### Example 10. Production of L-proline by E. coli 702ilvA-ΔleuO.

To test the effect of inactivation of the *leuO* gene on L-proline production, DNA fragments from the chromosome of the above-described *E. coli* MG1655Δ *leuO::cat* can be transferred to the proline-producing *E. coli* strain 702ilvA by P1 transduction (Miller, J.H. (1972) Experiments in Molecular Genetics, Cold Spring Harbor Lab. Press, Plainview, NY). to obtain the strain 702ilvA-Δ*leuO*. The strain 702ilvA has been deposited in the Russian National Collection of Industrial Microorganisms (VKPM) (Russia, 117545 Moscow, 1 Dorozhny proezd, 1) on July 18, 2000 under accession number VKPM B-8012 and then converted to a deposit under the Budapest Treaty on May 18, 2001.

Both *E. coli* 702ilvA and 702ilvA-Δ*leuO* can be grown for 18-24 hours at 37 °C on L-agar plates. Then, these strains can be cultivated under the same conditions as in Example 7.

### Example 11. Production of L-arginine by E. coli 382-ΔleuO.

To test the effect of inactivation of the *leuO* gene on L-arginine production, DNA fragments from the chromosome of the above-described *E. coli* MG1655 Δ*leuO::cat* were transferred to the arginine-producing *E. coli* strain 382 by P1 transduction (Miller, J.H. (1972) Experiments in Molecular Genetics, Cold Spring Harbor Lab. Press, Plainview, NY) to obtain the strain *382-*Δ*leuO.* The strain 382 has been deposited in the Russian National Collection of Industrial Microorganisms (VKPM) (Russia, 117545 Moscow, 1 Dorozhny proezd, 1) on April 10, 2000 under accession number VKPM B-7926 and then converted to a deposit under the Budapest Treaty on May 18, 2001.

Both strains, 382*-*Δ*leuO* and 382, were each cultivated with shaking at 37 °C for 18 hours in a 3 ml of nutrient broth. 0.3 ml of the obtained cultures were inoculated into 3 ml of a fermentation medium in 20 x 200 mm test tubes, and cultivated at 32 °C for 48 hours on a rotary shaker.

After the cultivation, the amount of L-arginine which had accumulated in the medium was determined by paper chromatography using following mobile phase: butanol : acetic acid : water = 4 : 1 : 1 (v/v). A solution (2%) of ninhydrin in acetone was used as a visualizing reagent. A spot containing L-arginine was cut out, L-arginine was eluted in 0.5 % water solution of CdCl₂, and the amount of L-arginine was estimated spectrophotometrically at 540 nm. The results of 10 independent test tube fermentations are shown in Table 4.

The composition of the fermentation medium (g/l) was as follows:

| | |
|---|---|
| Glucose | 48.0 |
| (NH4)₂SO₄ | 35.0 |
| KH₂PO₄ | 2.0 |
| MgSO₄·7H₂O | 1.0 |
| Thiamine HCl | 0.0002 |
| Yeast extract | 1.0 |
| L-isoleucine | 0.1 |
| CaCO₃ | 5.0 |

Glucose and magnesium sulfate were sterilized separately. CaCO₃ was sterilized by dry-heat at 180°C for 2 hours. pH was adjusted to 7.0.

**Table 4**

| Strain | OD₅₄₀ | Amount of L-arginine, g/l |
|---|---|---|
| 382 | 18.6 ± 5.0 | 9.4 ± 0.8 |
| 382-Δl*euO* | 175 ± 1.0 | 10.5 ± 0.8 |

It can be seen from the Table 4, the strain 382-Δ*leuO* caused accumulation of a higher amount of L-arginine as compared with the strain 382.

### Industrial Applicability

According to the present invention, production of L-amino acid of a bacterium of the *Enterobacteriaceae* family can be enhanced, wherein said L-amino acid is selected from the group consisting of L-threonine, L-lysine, L-methionine, L-asparagine, L-aspartic acid, and L-arginine.

## Claims

1. A method for producing an L-amino acid comprising:
- cultivating an L-amino acid producing bacterium of the Enterobacteriaceae family, wherein said bacterium has been modified to attenuate expression of the leuO gene in a medium so as to produce and excrete said L-amino acid into the medium, and
- collecting said L-amino acid from the medium, wherein said L-amino acid is selected from the group consisting of L-threonine, L-lysine, L-methionine, L-asparagine, L-aspartic acid, and L-arginine,

2. The method according to claim 1, wherein said expression of the leuO gene is attenuated by inactivation of the leuO gene.

3. The method according to claim 1, wherein said bacterium belongs to the genus Escherichia.

4. The method according to claim 1, wherein said bacterium belongs to the genus Pantoea.

## Revendications

1. Procédé pour produire un acide L-aminé comprenant :
- la culture d'une bactérie produisant un acide L-aminé de la famille *Enterobacteriaceae,* dans laquelle ladite bactérie a été modifiée pour atténuer l'expression du gène leuO dans un milieu de sorte à produire et excréter ledit acide L-aminé dans le milieu, et
- la collecte dudit acide L-aminé depuis le milieu, dans laquelle ledit acide L-aminé est choisi dans le groupe constitué de la L-thréonine, la L-lysine, la L-méthionine, la L-asparagine, l'acide L-aspartique, et la L-arginine.

2. Procédé selon la revendication 1, dans lequel ladite expression du gène leuO est atténuée par inactivation du gène leuO.

3. Procédé selon la revendication 1, dans lequel ladite bactérie appartient au genre *Escherichia*

4. Procédé selon la revendication 1, dans lequel ladite bactérie appartient au genre *Pantoea*.

## Patentansprüche

1. Verfahren zur Herstellung einer L-Aminosäüre, umfassend:
- Kultivieren eines L-Aminosäure-produzierenden Bakteriums aus der Familie der Enterobacteriaceae, wobei das Bakterium modifiziert wurde, um die Expression des leuO-Gens abzuschwächen, in einem Medium, um die L-Aminosäure zu produzieren und in das Medium auszuscheiden und
- Gewinnen der L-Aminosäure aus dem Medium, wobei die L-Aminosäure ausgewählt ist aus der Gruppe bestehend aus L-Threonin, L-Lysin, L-Methionin, L-Asparagin, L-Asparaginsäure und L-Arginin.

2. Verfahren gemäß Anspruch 1, wobei die Expression des leuO-Gens mittels Inaktivierung des leuO-Gens abgeschwächt ist.

3. Verfahren gemäß Anspruch 1, wobei das Bakterium zum Genus Escherichia gehört.

4. Verfahren gemäß Anspruch 1, wobei das Bakterium zum Genus Pantoea gehört.


### SEQUENCE LISTING

<110> Ajinomoto Co., Inc.
<120> A METHOD FOR PRODUCING AN L-AMINO ACID USING A BACTERIUM OF THE ENTEROBACTERIACEAE FAMILY HAVING EXPRESSION OF THE leuO GENE ATTENUATED
<130> C456-C6044
<150> RU2005106346
   <151> 2005-03-10
<150> US60/723925
   <151> 2005-10-06
<160> 6
<170> PatentIn version 3.1
<210> 1
   <211> 1122
   <212> DNA
   <213> Escherichia coli
<220>
   <221> CDS
   <222> (1)..(1122)
<400> 1
   atg act cat tcc acg gca atg gat tct gtt ttt atc aga acc cgt atc         48
   Met Thr His Ser Thr Ala Met Asp Ser Val Phe Ile Arg Thr Arg Ile
   1               5                   10                  15
   ttt atg ttt tcc gaa ttt tac tca ttt tgc ttt ttc tta ttt tat atg         96
   Phe Met Phe Ser Glu Phe Tyr Ser Phe Cys Phe Phe Leu Phe Tyr Met
               20                  25                  30
   cat gat aaa tca tat tct tca gga tta ttt ctc tgc att cca ata agg        144
   His Asp Lys Ser Tyr Ser Ser Gly Leu Phe Leu Cys Ile Pro Ile Arg
           35                  40                  45
   gaa agg gag tta agt gtg aca gtg gag tta agt atg cca gag gta caa        192
   Glu Arg Glu Leu Ser Val Thr Val Glu Leu Ser Met Pro Glu Val Gln
       50                  55                  60
   aca gat cat cca gag acg gcg gag tta agc aaa cca cag cta cgc atg        240
   Thr Asp His Pro Glu Thr Ala Glu Leu Ser Lys Pro Gln Leu Arg Met
   65                  70                  75                  80
   gtc gat ctc aac tta tta acc gtt ttc gat gcc gtg atg cag gag caa        288
   Val Asp Leu Asn Leu Leu Thr Val Phe Asp Ala Val Met Gln Glu Gln
                   85                  90                  95
   aac att act cgt gcc gct cat gtt ctg gga atg tcg caa cct gcg gtc        336
   Asn Ile Thr Arg Ala Ala His Val Leu Gly Met Ser Gln Pro Ala Val
               100                 105                 110
   agt aac gct gtt gca cgc ctg aag gtg atg ttt aat gac gag ctt ttt        384
   Ser Asn Ala Val Ala Arg Leu Lys Val Met Phe Asn Asp Glu Leu Phe
           115                 120                 125
   gtt cgt tat ggc cgt ggt att caa ccg act gct cgc gca ttt caa ctt       432
   Val Arg Tyr Gly Arg Gly Ile Gln Pro Thr Ala Arg Ala Phe Gln Leu
       130                 135                 140
   ttt ggt tca gtt cgt cag gca ttg caa cta gta caa aat gaa ttg cct       480
   Phe Gly Ser Val Arg Gln Ala Leu Gln Leu Val Gln Asn Glu Leu Pro
   145                 150                 155                 160
   ggt tca ggt ttt gaa ccc gcg agc agt gaa cgt gta ttt cat ctt tgt       528
   Gly Ser Gly Phe Glu Pro Ala Ser Ser Glu Arg Val Phe His Leu Cys
                   165                 170                 175
   gtt tgc agc ccg tta gac agc att ctg acc tcg cag att tat aat cac       576
   Val Cys Ser Pro Leu Asp Ser Ile Leu Thr Ser Gln Ile Tyr Asn His
               180                 185                 190
   att gag cag att gcg cca aat ata cat gtt atg ttc aag tct tca tta       624
   Ile Glu Gln Ile Ala Pro Asn Ile His Val Met Phe Lys Ser Ser Leu
           195                 200                 205
   aat cag aac act gaa cat cag ctg cgt tat cag gaa acg gag ttt gtg       672
   Asn Gln Asn Thr Glu His Gln Leu Arg Tyr Gln Glu Thr Glu Phe Val
       210                 215                 220
   att agt tat gaa gac ttc cat cgt cct gaa ttt acc agc gta cca tta       720
   Ile Ser Tyr Glu Asp Phe His Arg Pro Glu Phe Thr Ser Val Pro Leu
   225                 230                 235                 240
   ttt aaa gat gaa atg gtg ctg gta gcc agc aaa aat cat cca aca att       768
   Phe Lys Asp Glu Met Val Leu Val Ala Ser Lys Asn His Pro Thr Ile
                   245                 250                 255
   aag ggc ccg tta ctg aaa cat gat gtt tat aac gaa caa cat gcg gcg       816
   Lys Gly Pro Leu Leu Lys His Asp Val Tyr Asn Glu Gln His Ala Ala
               260                 265                 270
   gtt tcg ctc gat cgt ttc gcg tca ttt agt caa cct tgg tat gac acg       864
   Val Ser Leu Asp Arg Phe Ala Ser Phe Ser Gln Pro Trp Tyr Asp Thr
           275                 280                 285
   gta gat aag caa gcc agt atc gcg tat cag ggc atg gca atg atg agc       912
   Val Asp Lys Gln Ala Ser Ile Ala Tyr Gln Gly Met Ala Met Met Ser
       290                 295                 300
   gta ctt agc gtg gtg tcg caa acg cat ttg gtc gct att gcg ccg cgt       960
   Val Leu Ser Val Val Ser Gln Thr His Leu Val Ala Ile Ala Pro Arg
   305                 310                 315                 320
   tgg ctg gct gaa gag ttc gct gaa tcc tta gaa tta cag gta tta ccg      1008
   Trp Leu Ala Glu Glu Phe Ala Glu Ser Leu Glu Leu Gln Val Leu Pro
                   325                 330                 335
   ctg ccg tta aaa caa aac agc aga acc tgt tat ctc tcc tgg cat gaa      1056
   Leu Pro Leu Lys Gln Asn Ser Arg Thr Cys Tyr Leu Ser Trp His Glu
               340                 345                 350
   gct gcc ggg cgc gat aaa ggc cat cag tgg atg gaa gag caa tta gtc      1104
   Ala Ala Gly Arg Asp Lys Gly His Gln Trp Met Glu Glu Gln Leu Val
   355 360 365
   tca att tgc aaa cgc taa 1122
   Ser Ile Cys Lys Arg
   370
<210> 2
   <211> 373
   <212> PRT
   <213> Escherichia coli
<400> 2
   Met Thr His Ser Thr Ala Met Asp Ser Val Phe Ile Arg Thr Arg Ile
   1               5                   10                  15
   Phe Met Phe Ser Glu Phe Tyr Ser Phe Cys Phe Phe Leu Phe Tyr Met
               20                  25                  30
   His Asp Lys Ser Tyr Ser Ser Gly Leu Phe Leu Cys Ile Pro Ile Arg
           35                  40                  45
   Glu Arg Glu Leu Ser Val Thr Val Glu Leu Ser Met Pro Glu Val Gln
       50                  55                  60
   Thr Asp His Pro Glu Thr Ala Glu Leu Ser Lys Pro Gln Leu Arg Met
   65                  70                  75                  80
   Val Asp Leu Asn Leu Leu Thr Val Phe Asp Ala Val Met Gln Glu Gln
                   85                  90                  95
   Asn Ile Thr Arg Ala Ala His Val Leu Gly Met Ser Gln Pro Ala Val
               100                 105                 110
   Ser Asn Ala Val Ala Arg Leu Lys Val Met Phe Asn Asp Glu Leu Phe
           115                 120                 125
   Val Arg Tyr Gly Arg Gly Ile Gln Pro Thr Ala Arg Ala Phe Gln Leu
       130                 135                 140
   Phe Gly Ser Val Arg Gln Ala Leu Gln Leu Val Gln Asn Glu Leu Pro
   145                 150                 155                 160
   Gly Ser Gly Phe Glu Pro Ala Ser Ser Glu Arg Val Phe His Leu Cys
                   165                 170                 175
   Val Cys Ser Pro Leu Asp Ser Ile Leu Thr Ser Gln Ile Tyr Asn His
               180                 185                 190
   Ile Glu Gln Ile Ala Pro Asn Ile His Val Met Phe Lys Ser Ser Leu
           195                 200                 205
   Asn Gln Asn Thr Glu His Gln Leu Arg Tyr Gln Glu Thr Glu Phe Val
       210                 215                 220
   Ile Ser Tyr Glu Asp Phe His Arg Pro Glu Phe Thr Ser Val Pro Leu
   225                 230                 235                 240
   Phe Lys Asp Glu Met Val Leu Val Ala Ser Lys Asn His Pro Thr Ile
                   245                 250                 255
   Lys Gly Pro Leu Leu Lys His Asp Val Tyr Asn Glu Gln His Ala Ala
               260                 265                 270
   Val Ser Leu Asp Arg Phe Ala Ser Phe Ser Gln Pro Trp Tyr Asp Thr
           275                 280                 285
   Val Asp Lys Gln Ala Ser Ile Ala Tyr Gln Gly Met Ala Met Met Ser
       290                 295                 300
   Val Leu Ser Val Val Ser Gln Thr His Leu Val Ala Ile Ala Pro Arg
   305                 310                 315                 320
   Trp Leu Ala Glu Glu Phe Ala Glu Ser Leu Glu Leu Gln Val Leu Pro
                   325                 330                 335
   Leu Pro Leu Lys Gln Asn Ser Arg Thr Cys Tyr Leu Ser Trp His Glu
               340                 345                 350
   Ala Ala Gly Arg Asp Lys Gly His Gln Trp Met Glu Glu Gln Leu Val
           355                 360                 365
   Ser Ile Cys Lys Arg
       370
<210> 3
   <211> 57
   <212> DNA
   <213> Artificial
<220>
   <223> primer
<400> 3
   ccagaggtac aaacagatca tccagagacg gcggagtagt aagccagtat acactcc 57
<210> 4
   <211> 57
   <212> DNA
   <213> Artificial
<220>
   <223> primer
<400> 4
   acgatcgagc gaaaccgccg catgttgttc gttatattaa gggcaccaat aactgcc 57
<210> 5
   <211> 19
   <212> DNA
   <213> Artificial
<220>
   <223> primer
<400> 5
   tgactcattc cacggcaat 19
<210> 6
   <211> 20
   <212> DNA
   <213> Artificial
<220>
   <223> primer
<400> 6
   ttagcgtttg caaattgaga 20